# FASCICULE DE BREVET EUROPEEN

(11) **EP 1 708 656 B1**
(45) Date de publication et mention de la délivrance du brevet: **10.04.2019**
(21) Numéro de dépôt: 05717418.7
(22) Date de dépôt: 14.01.2005
(51) Int. Cl.: A61F 5/00

(54) **CEINTURE GASTRIQUE**
MAGENBAND
GASTRIC BELT

(30) Priorité: 16.01.2004 FR 0400392
(43) Date de publication de la demande: 11.10.2006
(73) Titulaire: Medical Innovation Developpement, 69760 Limonest (FR)
(72) Inventeur: FRERING, Vincent, F-69005 Lyon (FR); DENIS, Pierre-André, F-69100 Villeurbanne (FR)
(74) Mandataire: Cabinet Plasseraud
(86) Numéro de dépôt international: PCT/FR2005/000083
(87) Numéro de publication internationale: WO 2005/072664

(56) Documents cités:
- EP-A- 0 769 282
- WO-A-00/00108
- FR-A- 2 799 118
- US-A- 5 152 770

## Description

L'invention concerne le domaine technique des dispositifs destinés à être implantés au niveau de la zone de raccordement entre la partie abdominale de l'oesophage et l'estomac, afin de créer une restriction locale permettant un contrôle de la quantité d'aliments ingérés par le patient porteur du dispositif.

Afin d'assurer cette restriction locale, il est connu de mettre en oeuvre une ceinture ou anneau gastrique gonflable, tel que, par exemple, décrit par les demandes de brevets EP 0 769 282, FR 2 799 118.

Selon ces documents, la ceinture gastrique gonflable comprend un corps tubulaire plat allongé, en matière souple, qui est, en partie au moins, élastiquement déformable et qui définit une chambre étanche gonflable pour présenter une face de travail destinée à être placée au contact de l'estomac et un dos à l'opposé de la face de travail.

La demande WO 00/00108 décrit, par ailleurs, une ceinture gastrique selon le préambule de la revendication 1.

Afin de permettre une fermeture de la ceinture en anneau, cette dernière comprend, également, des moyens de liaison équipant les extrémités du corps tubulaire et permettant de fermer la ceinture gastrique sous la forme d'un anneau, la face de travail étant, bien entendu, orientée vers l'intérieur. Enfin, la ceinture gastrique comprend, également, un cathéter de gonflage raccordé, de façon étanche, à la chambre gonflable et destiné à être raccordé à des moyens de gonflage. Les moyens de gonflage peuvent être, par exemple, constitués par un boîtier pourvu d'une membrane auto-obturable qui peut être transpercée par une aiguille de seringue ou analogue, au moyen de laquelle une injection ou un prélèvement de fluide, tel que, par exemple mais non nécessairement, du sérum physiologique, peut être effectué pour contrôler le gonflement de la chambre et ainsi les dimensions de l'étranglement stomacal réalisé au moyen de la ceinture gastrique gonflable.

Selon ces documents, le corps tubulaire et la chambre gonflable sont réalisés de manière à former, après fermeture de la ceinture et gonflage de cette dernière, un anneau de section régulière.

Or, si de telles bandes ont donné généralement satisfaction et ont permis, après leur implantation dans la plupart des cas, d'atteindre l'effet thérapeutique recherché, il a été observé, dans un nombre certes réduit de cas, des phénomènes de déplacement de la ceinture gonflable autour de l'oesophage ou de la partie supérieure de l'estomac, entraînant alors une inflammation des tissus au contact de la ceinture gastrique gonflable, pouvant justifier une ré-intervention de retrait de ladite ceinture.

Il est donc apparu le besoin de disposer d'un nouveau type de ceinture gastrique gonflable qui présente une plus grande stabilité d'implantation autour de l'estomac ou de l'oesophage, sans pour autant faire intervenir une suture de la ceinture gonflable sur la paroi stomacale ou oesophagienne.

Afin d'atteindre cet objectif, l'invention concerne une ceinture gastrique gonflable comprenant :
▪ un corps tubulaire allongé en matière souple qui est, en partie au moins, élastiquement déformable, qui définit une chambre étanche gonflable et qui présente un dos et une face de travail,
▪ des moyens de liaison disposés en relation avec les deux extrémités du corps tubulaire et permettant de fermer la ceinture gastrique sous la forme d'un anneau, la face de travail étant disposée à l'intérieur de l'anneau,
▪ un cathéter de gonflage raccordé, de façon étanche, à la chambre gonflable et destiné à être raccordé à des moyens de gonflage.

Selon l'invention, cette ceinture gastrique gonflable est caractérisée en ce que la paroi de la chambre, formant la face de travail, présente, dans un état dégonflé de la ceinture, une longueur supérieure ou égale à celle de la paroi de la chambre formant le dos, de manière que, lors de la fermeture de la ceinture en anneau et après gonflage, la paroi de la chambre, constituant la face de travail, forme des plis, chaque pli correspondant à une zone où la face de travail est rabattue ou repliée localement sur elle-même, de sorte que les régions de la face de travail adjacentes au pli et situées de part et d'autre du pli sont en contact.

Cette caractéristique de l'invention permet alors la formation de plis lors du gonflage de la ceinture, de sorte que l'intérieur de ladite ceinture ne présente, non pas la forme régulière d'un anneau mais, au contraire, une forme en étoile ou d'hypocycloïde irrégulière. Ces plis se forment de façon aléatoire et non prédéterminée, en fonction de la forme, des mouvements et de la résistance à la compression de la paroi de l'estomac. Ainsi, les plis sont susceptibles de se déplacer en cours d'utilisation de la ceinture, de sorte que les points de compression sur la paroi stomacale se déplacent également, évitant des nécroses ou des inflammations locales.

En effet, les inventeurs ont eu le mérite de mettre en évidence le fait qu'une telle forme irrégulière lors du gonflage garantissait une meilleure stabilité de la ceinture gastrique gonflable et, contrairement à une idée reçue, les zones de pincement qu'elle est éventuellement susceptible de créer au niveau de la paroi extérieure oesophagienne ou stomacale n'augmentent pas l'érosion ou les phénomènes d'inflammation de cette dernière. Cette caractéristique avantageuse de l'invention tient, certainement, au fait que le fluide de gonflage circule d'un compartiment, défini par les plis, à l'autre, assurant un équilibrage de la pression de gonflage et donc une meilleure répartition des efforts appliqués à l'estomac.

Toujours dans le même sens et selon une forme préférée mais non strictement nécessaire de l'invention, la ceinture gastrique gonflable est réalisée de manière que son corps tubulaire présente, dans un état dégonflé de la chambre et lorsque la ceinture n'est pas fermée, une forme sensiblement plane, sans pré-conformation. Dans cet état, qui pourrait alors être qualifié de repos, la bande gastrique gonflable présente une forme sensiblement de parallélépipède rectangle, abstraction faite des moyens de liaison, les parois de la chambre gonflable, en relation avec la face de travail et le dos, étant sensiblement planes et parallèles. Cette caractéristique de l'invention permet, d'une part, un positionnement aléatoire des plis lors du gonflage de la ceinture et, d'autre part, un déplacement de ces mêmes plis lors de l'application de contraintes à la ceinture par l'estomac.

Selon une caractéristique de l'invention, afin d'éviter d'éventuels problèmes de dégonflage au niveau des plis formés par la face de travail de la ceinture, la face interne de la paroi de la chambre comprend au moins une rainure de direction longitudinale destinée à définir un canal interne de circulation du fluide de gonflage au niveau du ou des plis formés. Selon l'invention, la rainure longitudinale ne s'étend pas nécessairement sur toute la longueur de la chambre gonflable, mais sur une partie au moins de cette longueur et, par exemple mais non exclusivement, dans une région médiane de la chambre et sur une longueur supérieure ou égale à la moitié de la longueur de la chambre gonflable.

Selon une forme préférée mais non strictement nécessaire, la face interne de la paroi de la chambre gonflable comprend au moins une série de rainures longitudinales parallèles.

Selon l'invention, cette rainure ou cette série de rainures peut être réalisée en tout endroit de la paroi de la chambre gonflable. Toutefois, de manière préférée, la ou les rainures longitudinales seront aménagées sur la face interne de la chambre gonflable correspondant au dos de la ceinture.

De manière préférée mais non strictement nécessaire, la face interne de la chambre gonflable, correspondant au dos de la ceinture, comprend alors deux séries de rainures longitudinales parallèles, chaque série étant située à proximité d'un bord du dos de la ceinture.

Selon une autre caractéristique de l'invention et toujours en vue d'augmenter encore la stabilité de la ceinture lors de son gonflage, la paroi de la chambre formant la face de travail comprend des moyens de limitation locale de l'élasticité de la paroi.

Dans une forme préférée mais non strictement nécessaire de réalisation, les moyens de limitation locale de l'élasticité occupent une région longitudinale médiane de la face de travail, de manière que, lors du gonflement de la ceinture, les bords longitudinaux de la face de travail se dilatent plus que la région médiane de ladite face de travail. Ainsi, les moyens de limitation locale de l'élasticité sont adaptés pour que le rayon de courbure de la région médiane de la face de travail lors du gonflage de la ceinture soit, autant que faire se peut, supérieur au rayon de courbure des régions latérales.

Selon l'invention, les moyens de limitation locale de l'élasticité de la paroi de la chambre gonflable peuvent être réalisés de toute façon appropriée, telle que, par exemple, sous la forme d'éléments rapportés, de préférence mais non nécessairement, élastiquement déformables et fixés ou insérés dans la paroi de la chambre gonflable.

Selon une forme préférée de réalisation, les moyens de limitation locale de l'élasticité comprennent une surépaisseur locale de la paroi de la chambre constitutive de la face de travail de la ceinture.

Selon encore une autre caractéristique de l'invention, chaque bord longitudinal de la chambre de gonflage est, de préférence mais non nécessairement, situé à distance du bord longitudinal correspondent du corps de la bande gastrique.

Cette caractéristique de l'invention contribue, également, à une plus grande stabilité de la ceinture gastrique lors de son implantation et après gonflage de cette dernière. De manière préférée mais non strictement nécessaire, chaque bord longitudinal de la chambre gonflable est alors situé à une distance, du bord longitudinal correspondent du corps de la ceinture, comprise entre 0,50 mm et 2,50 mm et, de préférence, entre 0,65 mm et 0,90 mm.

Selon une autre caractéristique de l'invention, la paroi de la chambre gonflable, formant le dos de la ceinture, comprend au moins une armature longitudinale souple inextensible, de manière à favoriser, lorsque la ceinture est fermée en anneau et lors du gonflage de la chambre, une déformation centripète de la chambre gonflable.

De manière préférée mais non strictement nécessaire, l'armature inextensible est alors complètement entourée du matériau constitutif du corps de la ceinture. Ainsi, la ceinture est moulée en une seule injection pour former une seule pièce autour de l'armature inextensible, sans autres éléments rapportés que des bouchons au niveau des points d'injection et, éventuellement, des éléments constitutifs des moyens de liaison pour la fermeture en anneau de la ceinture.

Par ailleurs, selon l'invention, les moyens de liaison, permettant la fermeture de la ceinture sous la forme d'un anneau autour de l'oesophage ou de la partie supérieure de l'estomac, peuvent être réalisés de toute façon appropriée. Toutefois, de manière préférée mais non strictement nécessaire, les moyens de liaison comprennent :
▪ en relation avec une première extrémité, dite de gonflage du corps, une queue de raccordement du cathéter à la chambre gonflable,
▪ et, en relation avec l'extrémité opposée dite libre du corps, au moins un arceau de réception de la queue de raccordement.

Selon une caractéristique de l'invention, la queue de raccordement comprend au moins des moyens de verrouillage anti-retour destinés à coopérer avec l'arceau.

Selon l'invention, les moyens de verrouillage anti-retour peuvent être réalisés de toute façon appropriée. Selon une forme de réalisation préférée mais non strictement nécessaire, les moyens de verrouillage comprennent au moins une conformation en sapin ou lancéolée.

Selon une autre caractéristique de l'invention, l'arceau est disposé au dos de la ceinture, de manière à éviter les risques de blessure de la paroi oesophagienne ou stomacale.

Selon une caractéristique de l'invention, les moyens de liaison comprennent au moins deux arceaux alignés. De manière préférée, l'arceau, situé le plus près de l'extrémité libre du corps, présente alors une forme évasée vers l'extrémité libre de la ceinture et convergeant vers le deuxième anneau, de façon à assurer le guidage du cathéter vers le deuxième arceau lors de la procédure de fermeture de la ceinture.

De manière préférée, afin d'assurer un guidage optimal, la largeur de l'arceau est alors supérieure à 6 mm.

Selon une caractéristique de l'invention, afin d'éviter une déviation des deux extrémités du corps lorsque la ceinture est fermée, l'anneau le plus proche de l'extrémité libre est situé à une distance de l'extrémité libre inférieure à 5 mm et, de préférence, inférieur à 3 mm, cette distance étant mesurée entre un plan transversal passant par le sommet de l'arceau au niveau de sa partie la plus proche de l'extrémité libre et un plan parallèle passant par cette extrémité libre.

Selon encore une autre caractéristique de l'invention, la face interne de l'arceau présente des picots ou des stries parallèles à l'axe longitudinal du corps et au sens d'introduction du cathéter et de la queue de verrouillage, de manière à réduire les frottements lors du passage du cathéter et de la queue.

Selon une autre caractéristique et toujours en vue de faciliter le passage du cathéter, ce dernier est, de préférence mais non nécessairement, recouvert d'un produit à faible coefficient de friction, tel que, par exemple, du *téflon.*

Selon une autre caractéristique de l'invention, l'une au moins des extrémités de la chambre gonflable est située à distance de l'extrémité correspondante du corps pour définir une extrémité renforcée de préhension.

La mise en oeuvre d'une telle extrémité renforcée permet de définir une zone particulièrement appropriée à la prise en charge de la ceinture gastrique par une pince, sans risque de perforation de la paroi de la chambre gonflable.

Dans une forme de réalisation, l'extrémité de la chambre gonflable est alors située à une distance de l'extrémité correspondante du corps supérieure à 5 mm et, de préférence, supérieure à 7 mm et, de manière plus particulièrement préférée, à une distance comprise entre 7 mm et 15 mm, 10 mm étant un compromis avantageux. Il est ainsi fourni une extrémité pleine de préhension.

Dans une autre forme de réalisation, le renfort de l'extrémité est obtenu en adoptant, dans la zone d'extrémité, entre les trois côtés de la chambre gonflable et les bords correspondants du corps de la bande, une distance supérieure à celle adoptée pour le reste de la bande. De manière préférée mais non exclusive, cette distance, dans la zone d'extrémité, sera supérieure à 0,75 mm et, de manière plus particulièrement préférée, comprise entre 0,75 mm et 2,50 mm, une distance comprise entre 1,50 mm et 2,50 mm offrant un bon compromis.

Dans le même sens, selon une autre caractéristique de l'invention, la paroi de la chambre gonflable est renforcée à proximité de l'extrémité libre du corps de la ceinture et présente, à cet effet, une surépaisseur locale dans cette région.

Selon encore une autre caractéristique de l'invention, la ceinture comprend des moyens de repérage optique du dos et/ou de la face de travail du corps. Selon une forme de réalisation préférée, les moyens de repérage comprennent des signes optiques apposés sur le dos de la ceinture, ainsi que sur la face correspondante du cathéter.

Selon une autre caractéristique de l'invention, la ceinture comprend des moyens d'indication optiques de la direction de l'extrémité libre du cathéter et/ou de l'extrémité libre du corps de la ceinture. Selon une forme de réalisation, ces moyens d'indication optique comprennent des flèches ou des triangles dont une pointe est orientée vers l'extrémité libre du cathéter.

De tels moyens de repérage et/ou d'indication optique facilitent alors le travail du chirurgien dans le cadre d'une implantation par voie scélioscopique.

Selon encore une autre caractéristique de l'invention, l'extrémité libre du cathéter, située à l'opposé du corps de la ceinture, est obturée au moyen d'un bouchon de forme tronconique qui, d'une part, empêche l'introduction, de matière organique, dans le cathéter lors de la mise en place de la ceinture et, d'autre part, facilite l'introduction du cathéter dans le ou les arceaux constitutifs des moyens de liaison.

Bien entendu, les différentes caractéristiques de l'invention, évoquées ci-dessus, peuvent être mises en oeuvre toutes ensembles ou, en partie seulement, selon différentes combinaisons pour la réalisation d'une ceinture gastrique gonflable conforme à l'invention.

Par ailleurs, diverses autres caractéristiques de l'invention ressortent de la description ci-dessous effectuée en référence aux dessins annexés qui illustrent une forme préférée, mais non limitative, de réalisation d'une ceinture gastrique gonflable selon l'invention.
La **fig. 1** est une perspective dans un état de repos d'une ceinture gastrique selon l'invention.
La **fig. 2** est une coupe longitudinale selon le plan **II-II** de la **fig. 1****.**
La **fig. 3** est une coupe transversale selon la ligne **III-III** de la **fig. 2****.**
La **fig. 4** est une vue de la ceinture gastrique gonflable selon la **fig. 1** dans un état fermé en anneau et semi-gonflé.
La **fig. 5** est une coupe partielle, selon la ligne **V-V** de la **fig.** 2.
La **fig. 6** est une coupe, analogue à la **fig. 3****,** dans un état gonflé de la ceinture.
La **fig.** 7 est une coupe, analogue à la **fig. 3****,** montrant une variante de réalisation d'une extrémité renforcée de la ceinture.
La **fig. 8** est une coupe partielle selon la ligne **VIII-VIII** de la **fig. 7****.**

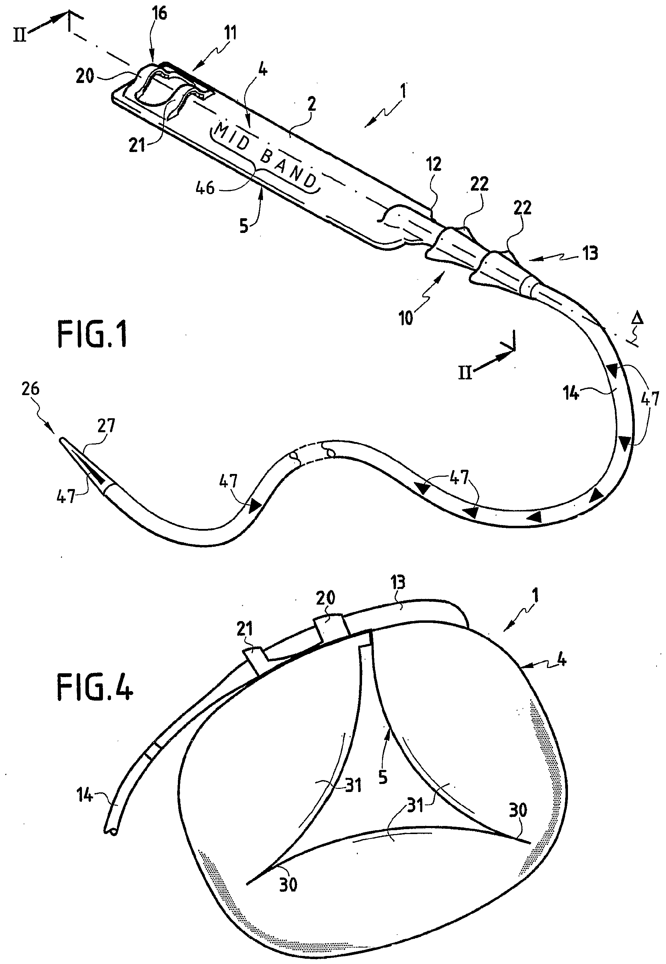

Une ceinture gastrique selon l'invention, illustrée aux **fig. 1** à **3** et désignée dans son ensemble par la référence **1,** comprend un corps tubulaire **2** qui définit au moins une chambre étanche gonflable **3.** Selon l'exemple illustré, le corps **2** présente, en élévation en vue de dessus, une forme générale rectangulaire, qui correspond à une forme préférée de réalisation, sans toutefois constituer la seule forme qui puisse être adoptée pour le corps **2.**

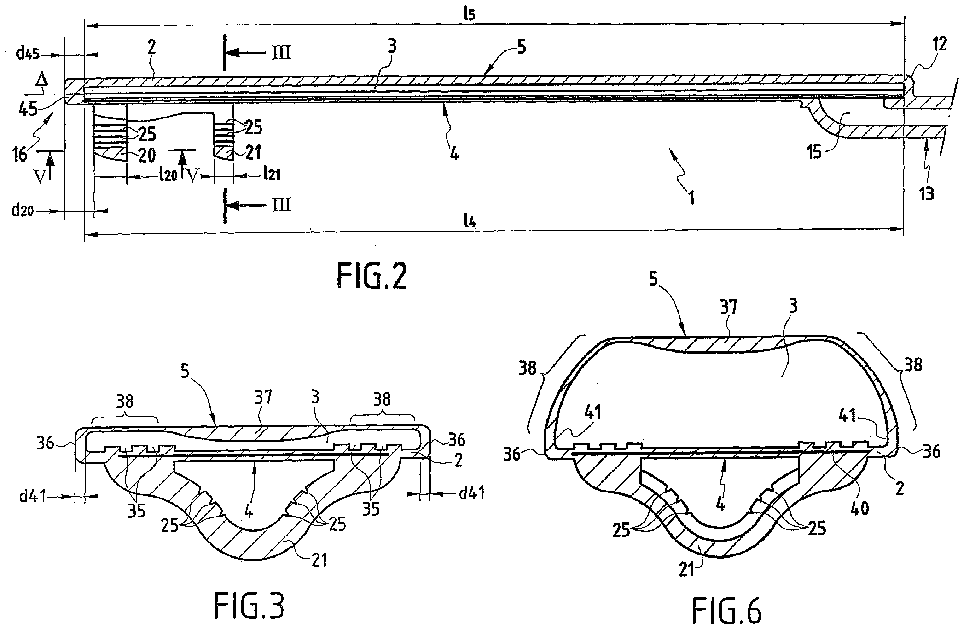

Dans un état dégonflé de la chambre **3,** il doit, par ailleurs, être considéré que le corps **2** présente une forme générale plate et une section droite transversale, telle que plus particulièrement illustrée à la **fig. 3****,** sensiblement rectangulaire, la chambre gonflable **3** présentant une section droite transversale de forme générale s'inscrivant également dans un rectangle.

La ceinture gastrique **1** présente alors un dos **4** et une face de travail **5,** destinée à venir au contact de la zone oesophagienne ou stomacale, au niveau de laquelle la ceinture sera placée, comme cela ressortira de la suite.

Afin de permettre une fermeture de la ceinture **1** en anneau, comme cela est illustré à la **fig. 4****,** la ceinture **1** comprend, également, des moyens de liaison **10** et **11** équipant les deux extrémités de la ceinture. Les moyens de liaison **10** et **11** peuvent être réalisés de toute façon appropriée.

Selon l'exemple illustré, les moyens de liaison **10** comprennent, tout d'abord, au niveau d'une première extrémité **12** du corps **2,** dite de gonflage, une queue de raccordement **13,** d'un cathéter **14,** à la chambre gonflable **3.**

Selon l'exemple illustré, la queue de raccordement **13** s'étend alors sensiblement dans le prolongement longitudinal du corps **2** et présente un canal interne **15** raccordé à la chambre **3.** Bien entendu, ce canal **15** communique avec le conduit interne du cathéter **14.**

Les moyens de liaison **11,** situés au niveau de l'extrémité opposée à l'extrémité de gonflage **12** et dite extrémité libre **16,** sont, selon l'exemple illustré, constitués par au moins un et, dans le cas présent, de deux arceaux **20, 21** disposés au niveau du dos **4** du corps **2** et destinés à recevoir la queue **13.**

Selon l'exemple illustré, les deux arceaux **20** sont situés à distance l'un de l'autre et la queue **13** comprend des moyens **22** de blocage ou anti-retours destinés à empêcher tout retrait intempestif de la queue de raccordement **13** après engagement de cette dernière dans les arceaux **20, 21.** Selon l'exemple illustré, les moyens de blocage anti-retour comprennent deux conformations **22** en sapin ou lancéolées, qui sont chacune destinées à coopérer avec un arceau **20, 21** correspondant.

Selon l'exemple illustré, l'arceau **20,** situé le plus près de l'extrémité libre **16** du corps **2,** présente une largeur l**₂₀**, mesurée parallèlement à l'axe longitudinal **Δ** du corps **2** et, au sommet de l'arceau **20,** supérieure à 5 mm. Cette disposition de l'invention permet ainsi au premier arceau d'assurer un guidage du cathéter **14** lors de la procédure de fermeture de la ceinture. Selon l'exemple illustré, la largeur **l₂₀** du premier arceau est supérieure à la largeur **l₂₁** du deuxième arceau.

De plus, toujours selon l'exemple illustré, afin de favoriser ce passage du cathéter et réduire, autant que faire se peut, les efforts nécessaires à ce geste chirurgical, les arceaux présentent, sur leur face interne, une série de stries **25** qui viennent réduire la surface de contact du cathéter avec l'arceau correspondant, de manière à réduire les forces de friction. Les stries **25** s'étendent parallèlement à l'axe longitudinal **Δ** du corps **2** et dans la direction d'introduction du cathéter **14.** Afin de réduire encore la force nécessaire au passage du cathéter **14,** il peut également être envisagé de recouvrir la surface extérieure du cathéter **14** d'un revêtement à faible coefficient de friction, tel que, par exemple, du téflon.

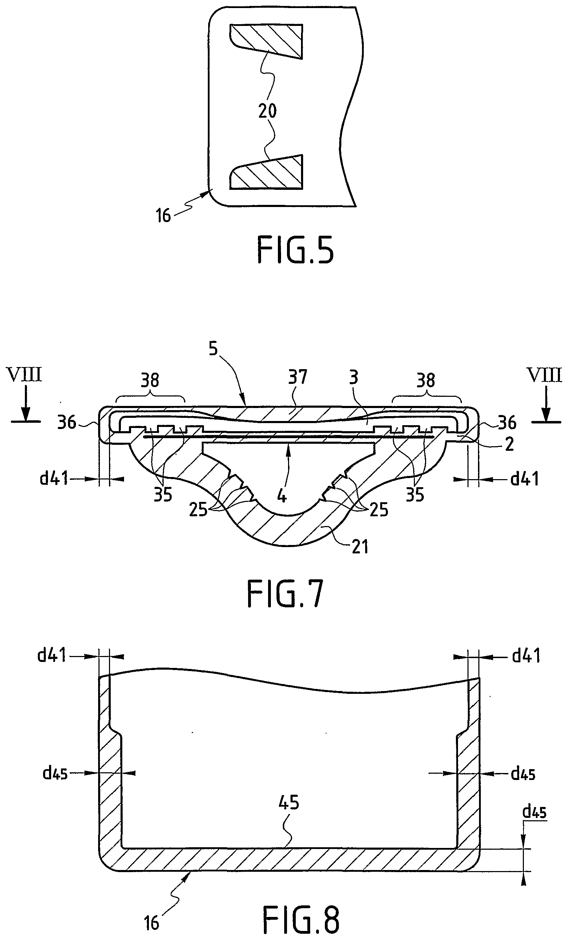

Toujours dans le sens d'une plus grande facilité d'introduction du cathéter **14,** le premier arceau **20** présente, de préférence, une forme évasée vers l'extrémité libre du corps **2** et convergeant vers le deuxième anneau **21,** comme illustré à la **fig. 5****.**

De même, l'extrémité libre **26** du cathéter **14** est obturée par un bouchon **27,** de forme conique, qui évite l'introduction de matières dans le canal du cathéter et facilite l'introduction du cathéter dans l'arceau **20** lors de la fermeture de la ceinture.

Le bouchon **27** sera sectionné après fermeture de la ceinture, pour la mise en place de moyens de gonflage, non représentés.

La ceinture gastrique gonflable **1** peut être réalisée en tout matériau biocompatible adapté, tel que, par exemple, du silicone biocompatible ou de grade implantable, qui confère au corps **2** la souplesse et l'élasticité nécessaire au gonflage de la chambre **3.**

Les moyens de liaison **10, 11** sont alors être mis en oeuvre pour fermer en anneau la ceinture gonflable **1,** comme cela est illustré à la **fig. 4****.**

Selon l'invention, le corps **2** est réalisé de manière que, lors du gonflage de la chambre **3,** il se forme des plis **30** conférant, à la section de passage définie par la ceinture **1** en anneau, une forme d'étoile irrégulière ou, encore, d'hypocycloïde également irrégulière, comme le montre plus particulièrement la **fig. 2****.** Chaque pli **30** correspond à une zone où la face de travail **5** est rabattue ou repliée localement sur elle-même, de sorte que les régions de la face de travail **5** adjacente au pli **30** et situées de part et d'autre du pli **30** sont en contact.

Afin d'atteindre cet objectif recherché de forme irrégulière ou aléatoire pour la section de passage définie par la ceinture gastrique **1** fermée en anneau et gonflée, le corps **2** est réalisé de manière que la longueur **l₅** de la paroi de la chambre gonflable **3,** formant la face de travail **5,** présente une longueur supérieure ou égale à la longueur **l₄** de la paroi de la chambre gonflable **3** formant le dos **4** de la ceinture **1** et cela dans un état dégonflé de ladite ceinture.

Selon l'exemple illustré, la longueur de la paroi **5** de la chambre gonflable **3** est sensiblement égale à celle de la paroi de la même chambre **3** constitutive du dos **4** de la bande de la ceinture gastrique **1.**

Selon la forme de réalisation illustrée, afin d'éviter des problèmes de dégonflage des différentes poches **31** formées par les plis **30,** la paroi interne de la chambre gonflable **3,** constitutive du dos **4,** présente deux séries de cannelures ou rainures de directions longitudinales **35** qui s'étendent, de préférence mais non nécessairement, sur toute la longueur de la paroi interne de la chambre gonflable **3.** Ces rainures **35** sont alors destinées à former des canaux au niveau des plis pour le passage du fluide de gonflage de la chambre **3.** Selon l'exemple illustré, les deux séries de rainures **35** sont placées à distance l'une de l'autre et chacune à proximité d'un bord longitudinal **36** du corps **2.**

Il doit être souligné que les plis **30** réalisés par la ceinture gonflable **1** selon l'invention contribuent à assurer une bonne stabilité de cette dernière au niveau de la paroi oesophagienne ou stomacale et délimitent les mouvements de roulement susceptibles d'induire une inflammation de ladite paroi stomacale ou oesophagienne.

Selon l'invention, afin d'augmenter encore cette stabilité, il est également prévu de mettre en oeuvre des moyens **37** de limitation locale de l'élasticité de la paroi de la chambre gonflable **3** constitutive de la face de travail **5.** Selon l'exemple illustré, ces moyens **37** de limitation de l'élasticité locale sont constitués par une zone longitudinale médiane de la paroi **5** qui présente une surépaisseur **37** par rapport à deux bandes latérales **38** de cette même paroi **5.** Ainsi, lors du gonflage de la chambre **3,** la dilatation de la paroi **5** intervient, de manière préférentielle, sur les bords **38** de la ceinture et la région médiane de la paroi **5** présente alors un rayon de courbure plus important que celui des bords latéraux ou zones latérales **38,** comme le montre la **fig. 6****.** Cette caractéristique avantageuse de l'invention contribue donc à la stabilité de la ceinture en réduisant la tendance au roulis de cette dernière.

Par ailleurs, selon une autre caractéristique de l'invention, afin d'assurer un meilleur contrôle de la restriction stomacale formée par la ceinture **1,** il est également prévu d'incorporer, dans la paroi du corps **2** constitutif du dos **4,** une armature **40** souple, inextensible. Ainsi, lors du gonflage de la chambre **3,** la ceinture **1** connaît une déformation essentiellement centripète. L'armature souple **40** peut être réalisée en tout matériau souple inextensible adapté, tel que, par exemple mais non exclusivement, un tissu de dacron.

De manière préférée, l'armature **40** se trouve complètement noyée dans la paroi du corps **2** formant le dos **4** et se trouve complètement entourée du matériau constitutif du corps **2.**

De plus, selon l'exemple illustré, il doit être remarqué que les bords longitudinaux internes **41** de la chambre gonflable **3** sont situés à une distance **d₄₁** supérieure à 0,5 mm et, de préférence, comprise entre 0,50 mm et 2,50 mm du bord longitudinal **36** correspondant du corps **2,** une distance comprise entre 0,65 mm et 0,90 mm offrant un bon compromis. Cette caractéristique contribue, également, à la stabilité de la ceinture **1** autour de la paroi oesophagienne ou stomacale.

Selon une autre caractéristique de l'invention, l'extrémité **45** de la chambre **3,** située du côté de l'extrémité libre **16** du corps **2,** se trouve également à distance de ladite extrémité et, de préférence, à une distance **d₄₅** supérieure à 5 mm et, de façon particulièrement préférée, comprise entre 7 mm et 15 mm et valant, selon l'exemple, 10 mm au moins, de manière à définir une zone pleine au niveau de laquelle il est possible de prendre la ceinture au moyen d'une pince, sans risque de perforation de la paroi de la chambre de gonflage **3.** Afin de renforcer encore la résistance à la perforation de l'extrémité de la ceinture, la paroi de la chambre gonflable présente une surépaisseur, au niveau de son extrémité orientée vers l'extrémité libre **16** du corps **2.**

Cette caractéristique de l'invention contribue à faciliter la mise en place de la ceinture gastrique au moyen d'outils de chirurgie coelioscopique.

Par ailleurs, selon l'exemple illustré, afin d'éviter une déviation des deux extrémités **12** et **16** du corps **2,** lorsque la ceinture **1** est fermée en anneau, l'arceau **20,** situé le plus près de l'extrémité libre **16,** se trouve placé à une distance **d₂₀** de l'extrémité **16** correspondante du corps **2** inférieure ou égale à 5 mm et, de préférence, inférieure à 3 mm.

Selon l'invention, afin de faciliter le travail du chirurgien utilisant la voie coelioscopique, il peut être mis en oeuvre des moyens **46** de repérage optique du dos ou face dorsale **4** et/ou de la face de travail **5** du corps **2.** Selon l'exemple illustré **fig. 1****,** ces moyens de repérage **46** comprennent des caractères d'identification de la ceinture apposés sur le dos **4,** tandis que la face de travail est vierge. Ainsi, l'observation par l'endoscope de ces indications informe le chirurgien de l'orientation du corps de la ceinture. Selon l'exemple illustré, les indications **46** sont complétées par une série de marques **47,** aménagées sur la face du cathéter **14** correspondant au dos **4** du corps **2.** Les marques **47** présentent chacune ici la forme d'un triangle dont un sommet est dirigé vers l'extrémité et orientée vers l'extrémité libre **26** du cathéter, de sorte qu'elles remplissent également une fonction de moyen d'indication optique de la direction de l'extrémité libre du cathéter. L'observation des marques **47** facilite donc au chirurgien la mise en place de la ceinture.

Selon l'exemple de réalisation décrit précédemment en relation avec la **fig. 2****,** l'extrémité libre renforcée **16** du corps de la ceinture est constitué par une zone pleine. Toutefois, au sens de l'invention, l'extrémité renforcée n'est pas nécessairement réalisée de cette manière. Ainsi, les **fig. 7** et **8** illustrent une autre forme de réalisation de cette extrémité renforcée.

Selon cette autre forme de réalisation, la distance **d₄₅**, séparant le bord intérieur de la chambre gonflable 3 du bord externe correspondant du corps **2,** est augmentée localement dans la région extrême du corps par rapport à la distance **d₄₁** séparant le bord interne de la chambre **3** du bord correspondant du corps **2** pour le reste de la bande, comme le montre la **fig. 8****.** Selon l'exemple illustré, la distance **d₄₅** est choisie pour être comprise entre 1,50 mm et 2,50 mm. Cette augmentation locale sur trois côtés réduit les risques de détérioration de la bande par une pince de préhension au niveau de l'extrémité libre.

De plus, selon l'exemple illustré, cette disposition est complétée par une augmentation locale d'épaisseur de la paroi constitutive de la face de travail **5,** à proximité de l'extrémité **16** du corps **2,** comme le montre la **fig. 7****.**

Bien entendu, diverses autres modifications peuvent être apportées à l'invention sans sortir de son cadre.

## Revendications

1. Ceinture gastrique gonflable comprenant :
▪ un corps tubulaire allongé (2) en matière souple qui est, en partie au moins, élastiquement déformable, qui définit une chambre étanche gonflable (3) et qui présente un dos (4) et une face de travail (5),
▪ des moyens de liaison (10, 11) disposés en relation avec les deux extrémités (12, 16) du corps tubulaire (2) et permettant de fermer la ceinture gastrique sous la forme d'un anneau, la face de travail (5) étant disposée à l'intérieur de l'anneau,
▪ un cathéter de gonflage raccordé, de façon étanche, à la chambre gonflable et destiné à être raccordé à des moyens de gonflage,
**caractérisée en ce que** la paroi de la chambre (3), formant la face de travail (5), présente, dans un état dégonflé de la ceinture, une longueur (15) supérieure ou égale à la longueur (14) de la paroi de la chambre (3) formant le dos (4), de manière que, lors de la fermeture de la ceinture en anneau et après gonflage, la paroi de la chambre, constituant la face de travail (5), forme des plis (30), chaque pli (30) correspondant à une zone où la face de travail (5) est rabattue ou repliée localement sur elle-même, de sorte que les régions de la face de travail (5) adjacentes au pli (30) et situées de part et d'autre du pli (30) sont en contact.

2. Ceinture gastrique gonflable selon la revendication 1, **caractérisée en ce que** la face interne de la paroi de la chambre (**3**) comprend au moins une rainure (**35**) de direction longitudinale, destinée à définir un canal interne de circulation du fluide de gonflage au niveau des plis (**30**).

3. Ceinture gastrique gonflable selon la revendication 2, **caractérisée en ce que** la face interne de la paroi de la chambre (**3**) comprend au moins une série de rainures longitudinales (**35**) parallèles.

4. Ceinture gastrique gonflable selon la revendication 2 ou 3, **caractérisée en ce que** la ou les rainures longitudinales sont aménagées sur la face interne de la chambre (**3**) correspondant au dos (**4**) de la ceinture.

5. Ceinture gastrique gonflable selon la revendication 1, **caractérisée en ce que** la face interne de la chambre, correspondent au dos de la ceinture, comprend deux séries de rainures longitudinales parallèles (**35**), chaque série étant située à proximité d'un bord (**36**) du dos (**4**) de la ceinture.

6. Ceinture gastrique gonflable selon l'une des revendications 1 à 5, **caractérisée en ce que** la paroi de la chambre (**3**), formant la face de travail (**5**), comprend des moyens (**37**) de limitation locale de l'élasticité de la paroi (**5**).

7. Ceinture gastrique gonflable selon la revendication 6, **caractérisée en ce que** les moyens de limitation locale de l'élasticité (**37**) occupent une région longitudinale médiane de la face de travail, de manière que, lors du gonflement de la ceinture, les bords longitudinaux de la face de travail se dilatent plus que la région médiane de ladite face de travail.

8. Ceinture gastrique gonflable selon la revendication 6 ou 7, **caractérisée en ce que** les moyens de limitation locale de l'élasticité (**37**) comprennent une surépaisseur locale de la paroi de la chambre (**3**) constitutive de la face de travail de la ceinture (**5**).

9. Ceinture gastrique gonflable selon l'une des revendications 1 à 8, **caractérisée en ce que** chaque bord longitudinal (**44**) de la chambre gonflable (**3**) est situé à distance du bord longitudinal (**36**) correspondant du corps (**2**) de la bande gastrique gonflable.

10. Ceinture gastrique gonflable selon la revendication 9, **caractérisée en ce que** chaque bord longitudinal de la chambre gonflable est situé à une distance (**d₄₁**) du bord longitudinal correspondant du corps, comprise entre 0,50 mm et 2 mm.

11. Ceinture gastrique gonflable selon l'une des revendications 1 à 10, **caractérisée en ce que** la paroi de la chambre gonflable (**3**), formant le dos (**4**) de la ceinture, comprend au moins une armature longitudinale souple (**40**) inextensible, de manière à favoriser, lorsque la ceinture est fermée en anneau et lors du gonflage, une déformation centripète de la chambre (**3**).

12. Ceinture gastrique gonflable selon la revendication 11, **caractérisée en ce que** le corps (2) est moulé en une injection autour de l'armature inextensible (**40**) et forme un ensemble monobloc qui entoure complètement ladite armature inextensible (**40**).

13. Ceinture gastrique gonflable selon l'une des revendications 1 à 12, **caractérisée en ce que** les moyens de liaison (**10**, **11**) comprennent :
a. en relation avec une première extrémité (**12**), dite de gonflage du corps (**2**), une queue de raccordement (**13**) du cathéter (**14**) à la chambre gonflable (**3**),
b. et, en relation avec l'extrémité opposée (**16**) dite libre du corps (**2**), au moins un arceau (**20**) de réception de la queue de raccordement (**13**).

14. Ceinture gastrique gonflable selon la revendication 13, **caractérisée en ce que** la queue de raccordement (**13**) comprend au moins des moyens (**22**) de verrouillage anti-retour destinés à coopérer avec l'arceau (**20**).

15. Ceinture gastrique gonflable selon la revendication 14, **caractérisée en ce que** les moyens de verrouillage anti-retour comprennent au moins une conformation (**22**) en sapin ou flèche.

16. Ceinture gastrique gonflable selon l'une des revendications 13 à 15, **caractérisée en ce que** l'arceau (**20**) est disposé au niveau du dos de la ceinture.

17. Ceinture gastrique gonflable selon l'une des revendications 13 à 16, **caractérisée en ce que** les moyens de liaison (**11**) comprennent au moins deux arceaux alignés (**20**, **21**), l'arceau (**20**) situé le plus près de l'extrémité libre (**16**) du corps (**2**) présentant une largeur (**l₂₀**), mesurée parallèlement à l'axe longitudinal du corps, supérieure à la largeur du deuxième arceau (**21**), de manière à définir un tunnel de guidage du cathéter (**14**) vers le deuxième arceau.

18. Ceinture gastrique selon l'une des revendications 13 à 17, **caractérisée en ce que** l'arceau (**20**) présente une largeur mesurée parallèlement à l'axe longitudinal du corps supérieure à 5 mm.

19. Ceinture gastrique selon l'une des revendications 13 à 18, **caractérisée en ce que** la face interne de l'arceau (**20**, **21**) présente des stries (**25**) parallèles à l'axe longitudinal (**Δ**) du corps (**2**), de manière à réduire les frottements lors du passage du cathéter.

20. Ceinture gastrique selon l'une des revendications 1 à 19, **caractérisée en ce que** le cathéter (**14**) est recouvert d'un produit à faible coefficient de friction.

21. Ceinture gastrique selon l'une des revendications 1 à 20, **caractérisée en ce que** l'une (**45**) au moins des extrémités de la chambre gonflable (**3**) est située à distance de l'extrémité (**16**) correspondante du corps (**2**) pour définir une extrémité renforcée de préhension.

22. Ceinture gastrique selon la revendication 21, **caractérisée en ce que** l'extrémité de la chambre gonflable est située à une distance (**d₄₅**), de l'extrémité correspondante du corps supérieure ou égale à 1,50 mm au niveau de l'extrémité renforcée.

23. Ceinture gastrique selon l'une des revendications 13 à 18, **caractérisée en ce que** l'arceau (**20**), situé à proximité de l'extrémité libre (**16**) du corps (**2**), est placé à une distance (**d₂₀**) inférieure à 5 mm et, de préférence, inférieure à 3 mm de ladite extrémité libre (**16**) du corps (**2**).

24. Ceinture gastrique selon l'une des revendications 1 à 23, **caractérisée en ce qu'**elle comprend des moyens (**46**) de repérage du dos (**4**) et/ou de la face de travail (**5**) du corps (**2**).

25. Ceinture gastrique selon l'une des revendications 1 à 24, **caractérisée en ce qu'**elle comprend des moyens (**47**) d'indication de la direction de l'extrémité libre (**26**) du cathéter (**14**) et/ou de l'extrémité libre (**16**) du corps (**2**).

## Patentansprüche

1. Aufblasbares Magenband beinhaltend:
• einen länglichen rohrförmigen Körper (2) aus flexiblem Material, der teilweise an den Müttern elastisch verformbar ist, der eine aufblasbare wasserdichte Kammer (3) definiert und der einen Rücken (4) und eine Arbeitsfläche (5) aufweist,
• Verbindungsmittel (10, 11), die in Bezug auf die beiden Enden (12, 16) des Rohrkörpers (2) angeordnet sind,und das Schließen des Magenbandes in Form eines Rings ermöglicht, wobei die Arbeitsfläche (5) innerhalb des Rings angeordnet ist,
• einen Aufblaskatheter, der dichtend mit der aufblasbaren Kammer verbunden und dazu bestimmt ist, mit Aufblasmitteln verbunden zu werden,
**dadurch gekennzeichnet, dass** die Wand der Kammer (3), die die Arbeitsfläche (5) bildet, in einem entleerten Zustand eine Länge (15) aufweist, die größer oder gleich der Länge (14) der Wand der Kammer (3) ist, die die Rückseite (4) bildet, so dass beim Schließen des Riemens im geschlossenen Zustand des Rings und nach dem Aufblasen die Wand der Kammer, die die Arbeitsfläche (5) bildet, Form der Falten (30), wobei jede Falte (30) einem Bereich entspricht, in dem die Arbeitsfläche (5) heruntergeklappt oder lokal auf sich selbst gefaltet ist, so dass die an die Falte (30) angrenzenden Bereiche der Arbeitsfläche (5), die sich auf beiden Seiten der Falte (30) befinden, in Kontakt stehen.

2. Aufblasbares Magenband nach Anspruch 1, **dadurch gekennzeichnet, dass** die Innenseite der Wand der Kammer (3) mindestens eine Nut (35) in Längsrichtung aufweist, die dazu bestimmt ist, einen inneren Kanal für die Zirkulation der Aufblasflüssigkeit auf dem Niveau der Falten (30) zu definieren.

3. Aufblasbares Magenband nach Anspruch 2, **dadurch gekennzeichnet, dass** die Innenfläche der Wand der Kammer (3) mindestens eine Reihe von parallelen Längsnuten (35) umfasst.

4. Aufblasbares Magenband nach Anspruch 2 oder 3, **dadurch gekennzeichnet, dass** die Längsnut(n) auf der der Rückseite (4) des Gurtes entsprechenden Innenseite der Kammer (3) angeordnet sind.

5. Aufblasbares Magenband nach Anspruch 1, **dadurch gekennzeichnet, dass** die Innenseite der Kammer, die der Rückseite des Gurtes entspricht, zwei Serien von parallelen Längsnuten (35) aufweist, wobei jede Serie in der Nähe einer Kante (36) der Rückseite (4) des Gurtes angeordnet ist.

6. Aufblasbares Magenband nach einem der Ansprüche 1 bis 5, **dadurch gekennzeichnet, dass** die Wand der Kammer (3), die die Arbeitsfläche (5) bildet, Mittel (37) zum lokalen Begrenzen der Elastizität der Wand (5) umfasst.

7. Aufblasbares Magenband nach Anspruch 6, **dadurch gekennzeichnet, dass** die Mittel zur lokalen Begrenzung der Elastizität (37) einen mittleren Längsbereich der Arbeitsfläche einnehmen, so dass sich beim Aufblasen des Gurtes die Längskanten der Arbeitsfläche mehr als der mittlere Bereich der Arbeitsfläche dehnen.

8. Aufblasbares Magenband nach Anspruch 6 oder 7, **dadurch gekennzeichnet, dass** die Mittel zur lokalen Begrenzung der Elastizität (37) eine lokale Übergröße der Wand der Kammer (3) umfassen, die die Arbeitsfläche des Gürtels (5) bildet.

9. Aufblasbares Magenband nach einem der Ansprüche 1 bis 8, **dadurch gekennzeichnet, dass** jede Längskante (44) der aufblasbaren Kammer (3) in einem Abstand von der entsprechenden Längskante (36) des Körpers (2) des aufblasbaren Magenbandes angeordnet ist.

10. Aufblasbares Magenband nach Anspruch 9, **dadurch gekennzeichnet, dass** jede Längskante der aufblasbaren Kammer in einem Abstand (d₄₁) von der entsprechenden Längskante des Körpers zwischen 0,50 mm und 2 mm angeordnet ist.

11. Aufblasbares Magenband nach einem der Ansprüche 1 bis 10, **dadurch gekennzeichnet, dass** die Wand der aufblasbaren Kammer (3), die den Rücken (4) des Gürtels bildet, mindestens eine flexible Längsverstärkung (40) umfasst, die nicht dehnbar ist, um beim Schließen des Gürtels in einem Ring und beim Aufblasen eine zentripetale Verformung der Kammer (3) zu fördern.

12. Aufblasbares Magenband nach Anspruch 11, **dadurch gekennzeichnet, dass** der Körper (2) zu einer Injektion um den unausdehnbaren Ring (40) geformt ist und eine Monoblockanordnung bildet, die die undehnbare Verstärkung (40) vollständig umschließt.

13. Aufblasbares Magenband nach einem der Ansprüche 1 bis 12, **dadurch gekennzeichnet, dass** die Verbindungsmittel (10, 11) umfassen:
a. in Verbindung mit einem ersten Ende (12), genannt das Körperaufblasende (2), einem Verbindungsschwanz (13) vom Katheter (14) zur aufblasbaren Kammer (3),
b. und in Verbindung mit dem gegenüberliegenden Ende (16) des Körpers (2) mindestens einen Aufnahmebügel (20) des Anschlussschweifs (13).

14. Aufblasbares Magenband nach Anspruch 13, **dadurch gekennzeichnet, dass** das Verbindungsende (13) mindestens Mittel (22) zum Verriegeln gegen Rückwärtsbewegung, die dazu bestimmt ist, mit dem Bogen (20) zusammenzuwirken, umfasst.

15. Aufblasbares Magenband nach Anspruch 14, **dadurch gekennzeichnet, dass** die Anti-Reverse-Verriegelungsmittel mindestens eine Konformation (22) aus Fichte oder Pfeil aufweisen.

16. Aufblasbares Magenband nach einem der Ansprüche 13 bis 15, **dadurch gekennzeichnet, dass** der Bogen (20) an der Rückseite des Gürtels angeordnet ist.

17. Aufblasbares Magenband nach einem der Ansprüche 13 bis 16, **dadurch gekennzeichnet, dass** die Verbindungsmittel (11) mindestens zwei ausgerichtete Reifen (20, 21) umfassen, wobei der Reifen (20) dem freien Ende (16) des Körpers (2) am nächsten ist, der eine Breite (l₂₀) aufweist, gemessen parallel zur Längsachse des Körpers, größer ist als die Breite des zweiten Bogens (21), um einen Führungstunnel vom Katheter (14) zum zweiten Bogen zu definieren.

18. Magenband nach einem der Ansprüche 13 bis 17, **dadurch gekennzeichnet, dass** der Bogen (20) eine parallel zur Längsachse des Körpers gemessene Breite von mehr als 5 mm aufweist.

19. Magenband nach einem der Ansprüche 13 bis 18, **dadurch gekennzeichnet, dass** die Innenfläche des Bogens (20, 21) Streifen (25) parallel zur Längsachse (A) des Körpers (2) aufweist, um die Reibung während des Durchtritts des Katheters zu reduzieren.

20. Magenband nach einem der Ansprüche 1 bis 19, **dadurch gekennzeichnet, dass** der Katheter (14) mit einem Produkt mit einem niedrigen Fraktionskoeffizienten abgedeckt ist.

21. Magenband nach einem der Ansprüche 1 bis 20, **dadurch gekennzeichnet, dass** eines (45) mindestens eines der Enden der aufblasbaren Kammer (3) in einem Abstand vom entsprechenden Ende (16) des Körpers (2) angeordnet ist, um ein verstärktes Griffende zu definieren.

22. Magenband nach Anspruch 21, **dadurch gekennzeichnet, dass** das Ende der aufblasbaren Kammer in einem Abstand (d₄₅) vom entsprechenden Ende des Körpers größer oder gleich 1,50 mm am verstärkten Ende angeordnet ist.

23. Magenband nach einem der Ansprüche 13 bis 18, **dadurch gekennzeichnet, dass** der Bogen (20), der sich in der Nähe des freien Endes (16) des Körpers (2) befindet, in einem Abstand (d₂₀) von weniger als 5 mm und vorzugsweise weniger als 3 mm von dem freien Ende (16) des Körpers (2) angeordnet ist.

24. Magenband nach einem der Ansprüche 1 bis 23, **dadurch gekennzeichnet, dass** er Mittel (46) zum Lokalisieren der Rückseite (4) und/oder der Arbeitsfläche (5) des Körpers (2) umfasst.

25. Magenband nach einem der Ansprüche 1 bis 24, **dadurch gekennzeichnet, dass** er Mittel (47) zum Anzeigen der Richtung des freien Endes (26) des Katheters (14) und/oder des freien Endes (16) des Körpers (2) umfasst.

## Claims

1. An inflatable gastric belt comprising:
• an elongated tubular body (2) made of flexible material which is, in part at least, elastically deformable, which defines a tight inflatable chamber (3) and which has a back (4) and a work face (5),
• connecting means (10,11) arranged relative to the two ends (12, 16) of the tubular body (2) and allowing the gastric belt to close in the form of a ring, the work face (5) being arranged inside the ring,
• an inflation catheter tightly connected to the inflatable chamber and intended to be connected to the inflation means,
**characterised in that** the wall of the chamber (3), forming the work face (5), presents, in a deflated state of the belt, a length (15) greater than or equal to the length (14) of the wall of the chamber (3) forming the back (4), such that, during closing of the ring belt and after inflation, the wall of the chamber, constituting the work face (5), forms folds (30), each fold (30) corresponding to an area where the work face (5) is locally turned down or folded on itself, so that regions of the work face (5) adjacent the fold (30) and situated on either sides of the fold (30) are in contact.

2. Inflatable gastric belt as claimed in Claim 1, **characterised in that** the internal face of the wall of the chamber (3) comprises at least one groove (35) of longitudinal direction, for defining an internal duct for circulation of the inflation fluid at the folds (30).

3. Inflatable gastric belt as claimed in Claim 2, **characterised in that** the internal face of the wall of the chamber (3) comprises at least one row of parallel longitudinal grooves (35).

4. Inflatable gastric belt as claimed in Claim 2 or 3, **characterised in that** the longitudinal groove or grooves are arranged on the internal face of the chamber (3) corresponding to the back (4) of the belt.

5. Inflatable gastric belt as claimed in Claim 1, **characterised in that** the internal face of the chamber, corresponding to the back of the belt comprises two rows of parallel longitudinal grooves (35), each row being situated near an edge (36) of the back (4) of the belt.

6. Inflatable gastric belt as claimed in any one of Claims 1 to 5, **characterised in that** the wall of the chamber (3), forming the work face (5), comprises means for local limitation (37) of the elasticity of the wall (5).

7. Inflatable gastric belt as claimed in Claim 6, **characterised in that** the means for local limitation of the elasticity (37) occupy a median longitudinal region of the work face, such that, during inflation of the belt, the longitudinal edges of the work face dilate more than the median region of said work face.

8. Inflatable gastric belt as claimed in Claim 6 or 7, **characterised in that** the means for local limitation of the elasticity (37) comprise local over-thickness of the wall of the chamber (3) making up the work face of the belt (5).

9. Inflatable gastric belt as claimed in any one of Claims 1 to 8, **characterised in that** each longitudinal edge (44) of the inflatable chamber (3) is situated at a distance from the corresponding longitudinal edge (36) of the body (2) of the inflatable gastric belt.

10. Inflatable gastric belt as claimed in Claim 9, **characterised in that** each longitudinal edge of the inflatable chamber is situated at a distance (d₄₁) from the corresponding longitudinal edge of the body, between 0.50 mm and 2 mm.

11. Inflatable gastric belt as claimed in any one of Claims 1 to 10, **characterised in that** the wall of the inflatable chamber (3), forming the back (4) of the belt, comprises at least one longitudinal inextensible and flexible fitting (40), such as to promote centripetal deformation of the chamber (3), when the belt is closed into a ring and during inflation.

12. Inflatable gastric belt as claimed in Claim 11, **characterised in that** the body (2) is injection-moulded around the inextensible fitting (40) and forms an monobloc assembly which completely encloses said inextensible fitting (40).

13. Inflatable gastric belt as claimed in any one of Claims 1 to 12, **characterised in that** the connecting means (10, 11) comprise :
• in relation to a first so-called inflation end (12) of the body (2), a connecting tail (13) of the catheter (14) to the inflatable chamber (3),
• and, in relation to the opposite so-called free end (16) of the body (2), at least one bow (20) for receiving the connecting tail (13).

14. Inflatable gastric belt as claimed in Claim 13, **characterised in that** the connecting tail (13) comprises at least anti-return locking means (22) for cooperating with the bow (20).

15. Inflatable gastric belt as claimed in Claim 14, **characterised in that** the anti-return locking means comprise at least a configuration (22) as a spruce tree or arrow.

16. Inflatable gastric belt as claimed in any one of Claims 13 to 15, **characterised in that** the bow (20) is arranged at the back of the belt.

17. Inflatable gastric belt as claimed in any one of Claims 13 to 16, **characterised in that** the connecting means (11) comprise at least two aligned bows (20, 21), the bow (20) situated the closest to the free end (16) of the body (2) having a width (l₂₀), measured parallel to the longitudinal axis of the body, greater than the width of the second bow (21), so as to define a tunnel for guiding the catheter (14) towards the second bow.

18. Gastric belt as claimed in any one of Claims 13 to 17, **characterised in that** the bow (20) has a width measured parallel to the longitudinal axis of the body greater than 5 mm.

19. Gastric belt as claimed in any one of Claims 13 to 18, **characterised in that** the internal face of the bow (20, 21) has stria (25) parallel to the longitudinal axis (Δ) of the body (2), so as to reduce friction as the catheter passes.

20. Gastric belt as claimed in any one of Claims 1 to 19, **characterised in that** the catheter (14) is covered in a product with low friction coefficient.

21. Gastric belt as claimed in any one of Claims 1 to 20, **characterised in that** one (45) at least of the ends of the inflatable chamber (3) is situated at a distance from the corresponding end (16) of the body (2) to define a reinforced prehension end.

22. Gastric belt as claimed in Claim 21, **characterised in that** the end of the inflatable chamber is situated at a distance (d₄₅) from the corresponding end of the body greater than or equal to 1.50 mm at the reinforced end.

23. Gastric belt as claimed in any one of Claims 13 to 18, **characterised in that** the bow (20), situated near the free end (16) of the body (2), is placed at a distance (d₂₀) of less than 5 mm and, preferably, less than 3 mm from said free end (16) of the body (2).

24. Gastric belt as claimed in any one of Claims 1 to 23, **characterised in that** it comprises means (46) for positioning the back (4) and/or the work face (5) of the body (2).

25. Gastric belt as claimed in any one of Claims 1 to 24, **characterised in that** it comprises means (47) for indicating the direction of the free end (26) of the catheter (14) and/or the free end (16) of the body (2).
